# FASCICULE DE BREVET EUROPEEN

(11) **EP 1 818 072 B1**
(45) Date de publication et mention de la délivrance du brevet: **09.09.2009**
(21) Numéro de dépôt: 06447020.6
(22) Date de dépôt: 09.02.2006
(51) Int. Cl.: A61M 25/06, A61M 5/32, A61M 25/00

(54) **Canule pour catheter d'hemodialyse**
Kanüle für Hämodialysekatheter
Cannula for hemodialysis catheter

(43) Date de publication de la demande: 15.08.2007
(73) Titulaire: Belgian Diagnostic Company S.A., 6900 Marche-en-Famenne (BE)
(72) Inventeur: Boogaerts, Marc, 01800 Charnoz-Sur-Ain (FR); Van Waeleghem, Jean-Pierre, 9840 De Pinte (BE)
(74) Mandataire: Cauchie, Daniel

(56) Documents cités:
- WO-A-03/092772
- DE-A1- 10 244 118
- US-A- 3 612 050
- US-A- 6 042 576
- US-A1- 2004 215 145

## Description

La présente invention se rapporte à une canule pour cathéter d'hémodialyse, du genre tube creux cylindrique, d'un seul tenant et entièrement en matière souple comprenant une extrémité distale et une extrémité proximale.

Le document US 6042576 décrit une canule veineuse et est considéré comme l'état de la technique la plus proche.

L'hémodialyse représente une technique d'épuration du sang par voie extra-rénale destinée à traiter l'insuffisance rénale à un stade de défaillance finale.

Selon cette technique, le sang est ponctionné du patient durant des séances de traitement de 4 à 5 heures avec un débit moyen de 250 ml/min, mis en contact avec un dialysat au travers d'une membrane semi-perméable synthétique par diffusion et ultrafiltration puis réintroduit dans le corps du patient. La communication avec les vaisseaux sanguins de celui-ci s'opère au moyen de deux canules de cathéter placées dans un environnement vasculaire approprié représenté généralement par une fistule artério-veineuse se définissant comme une anastomose d'une artère et d'une veine de voisinage.

La canule d'un cathéter d'hémodialyse représente, par conséquent, un moyen d'accès temporaire sous la forme d'un tube en matière souple muni d'une extrémité arrondie en relation avec la fistule d'hémodialyse et une extrémité proximale reliée au dialyseur. Cette canule peut être mise en place au moyen d'une aiguille ou trocart pourvue d'une extrémité pointue et tranchante. A cette fin, cette aiguille disposée dans la lumière de la canule est introduite dans la veine de large dimension, la canule est poussée dans celle-ci puis l'aiguille est retirée laissant cette canule en place pour permettre la ponction du sang artériel vicié ou sa ré-introduction après épuration. Toutefois, le placement d'une canule de cathéter d'hémodialyse s'avère généralement douloureux et son maintien pour la ponction de la fistule peut être traumatisant pour la paroi veineuse.

La présente invention a pour but de proposer une canule pour cathéter d'hémodialyse capable de pallier les inconvénients de l'état de la technique en ce qu'elle est apte à réduire la douleur et le traumatisme endoveineux lors de la canulation tout en modifiant favorablement le débit sanguin, c'est-à-dire en l'augmentant, avec pour conséquence une diminution de la durée d'une séance de dialyse.

Pour atteindre ce but, la canule de cathéter du type indiqué précédemment est caractérisée en ce que l'extrémité distale du tube comporte un biseau reliant deux points diamétralement opposés de sa paroi extérieure et formant un angle avec l'axe longitudinal de ce tube.

La canule selon l'invention est habituellement conçue en matière souple biocompatible de manière à éliminer les dangers d'extravasion et de dommage à la fistule d'hémodialyse. A ce titre, on peut faire appel à un polymère fluorocarboné en particulier le polytétrafluoroéthylène (PTFE) ou l'éthylène-propylène fluoré (FEP).

Avantageusement, l'angle formé par le biseau de cette canule est compris dans la gamme allant de 35° à 45°, un angle de 35° ou 45° étant préféré.

D'autre part, la canule selon l'invention comporte éventuellement des orifices latéraux, par exemple deux orifices notamment circulaires, situés à proximité de son extrémité distale. Ces orifices ont pour avantage, en particulier, d'assurer une régularisation du flux sanguin. La longueur d'une canule ainsi conçue varie généralement de 25 à 40 mm, sa lumière de 13 à 16 gauges et son diamètre extérieur de 1,2 à 2,4 mm.

Selon une autre caractéristique de l'invention, l'extrémité proximale de la canule est solidarisée à l'extrémité distale d'une embase, préférentiellement transparente, formant chambre de raccord habituellement sensiblement cylindrique dont l'extrémité proximale comporte des moyens de verrouillage à une tubulure extérieure ainsi qu'un orifice occupé par un obturateur.

Selon un mode de réalisation préféré, cette extrémité proximale présente, au niveau de sa paroi cylindrique extérieure, des moyens de verrouillage formés d'un raccord mâle capable de coopérer avec un raccord femelle au niveau de la tubulure, par exemple dans une fermeture de type Luer.

L'ensemble ainsi formé sera dénommé par la suite, aussi bien dans la description que dans les revendications, par le terme générique « cathéter ».

La chambre de raccord en question, habituellement en un matériau biologiquement compatible tel que le polyuréthane de polyéther, un copolymère éthylène/acétate de vinyle ou un copolymère éthylène/propylène fluoré comporte une zone de clampage capable de déformation élastique sous l'effet d'une pression. Cette zone, après écrasement, permet le retrait de l'obturateur sans perte de sang avant solidarisation de la chambre de raccord à un circuit extra-corporel.

Le cathéter ainsi conçu peut se présenter selon une configuration dans laquelle la chambre de raccord se situe dans le prolongement de la canule. Dans ce cas, ce cathéter sera dénommé « cathéter droit » ou cathéter biponction.

Toutefois, selon une autre caractéristique, le cathéter pourvu d'une canule selon l'invention peut comporter au niveau de sa chambre de raccord, une branche latérale formant un angle avec celle-ci, conférant à l'ensemble la forme d'un Y. Dans ce cas, un tel cathéter prendra la dénomination « cathéter en Y » ou cathéter uniponction.

Ce cathéter en Y peut résulter de l'adjonction latérale d'une branche supplémentaire au niveau de la chambre de raccord d'un cathéter droit ou d'une chambre de raccord formant un angle avec la canule. Dans l'un comme dans l'autre cas, la jonction de la branche latérale avec la chambre de raccord se situe entre l'extrémité distale de celle-ci et la zone de clampage.

En conséquence, selon une caractéristique supplémentaire de l'invention, la chambre de raccord est munie d'une branche latérale formant un angle avec celle-ci, cette branche comportant à son extrémité proximale, des moyens de verrouillage à une tubulure extérieure, ainsi qu'un orifice occupé par un obturateur amovible.

Selon un mode de réalisation préféré, cette extrémité proximale de la branche latérale présente, au niveau de sa paroi cylindrique extérieure, des moyens de verrouillage formés d'un raccord mâle capable de coopérer avec un raccord femelle au niveau de la tubulure par exemple dans une fermeture de type Luer.

Au surplus, la branche latérale comporte une zone de clampage capable de déformation élastique c'est-à-dire une zone analogue à la zone de clampage de la chambre de raccord.

Selon une autre caractéristique de l'invention, la canule comprend, logée dans sa lumière, une aiguille creuse formant trocart, aiguille dont l'extrémité distale est munie d'un biseau principal reliant deux points diamétralement opposés de sa paroi extérieure et formant un angle avec son axe longitudinal.

De manière particulièrement préférée, l'angle aigu formé par le biseau de la canule est supérieur à l'angle aigu formé par le biseau principal de l'aiguille, celui-ci se situant généralement et préférentiellement dans la gamme allant de 15° à 25° en particulier de 18° à 22°.

Au surplus, selon une autre caractéristique avantageuse de l'invention, l'angle aigu formé par le biseau de la canule et l'angle aigu formé par le biseau principal de l'aiguille sont ouverts dans le même sens ou sensiblement dans le même sens lors de la mise en place de ladite canule dans une fistule d'hémodialyse. Cette caractéristique s'avère particulièrement avantageuse pour faciliter la pénétration cutanée et veineuse de l'aiguille accompagnée de la canule.

Selon un mode de réalisation préféré de l'aiguille, les deux côtés de son biseau principal sont pourvus eux-mêmes de biseaux ou chanfreins latéraux secondaires qui délimitent la pointe aiguë de cette aiguille sur l'extrémité du biseau principal. Les faces de ces deuxième et troisième biseaux, à savoir la face du biseau de droite et celle du biseau de gauche, convergent vers l'intérieur à partir d'un point situé le long de la face du biseau principal jusqu'à un point terminal où leurs plans se rejoignent.

Avantageusement, ces deuxième et troisième biseaux occupent 40 à 60 % de la longueur totale du biseau principal.

L'aiguille - trocart, habituellement en acier inoxydable ayant subi un traitement siliconé, présente généralement un diamètre allant de 1,05 mm à 1,475 mm.

D'autre part, son extrémité proximale est solidarisée à l'extrémité distale d'une embase transparente ou chambre de visualisation habituellement en matériau synthétique tel que le polyuréthane et dont l'extrémité proximale est munie d'un bouchon amovible. Cette chambre est destinée à visualiser le reflux sanguin lors de la ponction.

Au surplus, l'extrémité distale de cette même chambre est pourvue d'un moyen anti-retrait capable de coopérer avec la zone de clampage de la chambre de raccord. Ce moyen anti-retrait, généralement sous la forme d'un clip anti-retrait, garantit une qualité de ponction indépendante de la dureté des tissus à pénétrer et permet la saisie du cathéter par sa partie centrale sans risque de retrait de l'aiguille.

Des tests comparatifs ont été effectués en vue d'étudier les débits sanguins réels obtenus durant une hémodialyse au moyen d'une canule selon l'invention ou d'une canule ordinaire.

### 1) Premier essai

Lors de cet essai, deux types de canules selon l'invention ont été utilisés comparativement à un type de canule classique. Les canules selon l'invention présentaient un biseau distal de 45° alors que la canule classique possédait une extrémité distale arrondie.

### Patients et matériels

Deux patients en stade terminal d'insuffisance rénale et traités par hémodialyse depuis au moins 6 mois ont été admis dans l'étude. Au début de celle-ci, la fistule artério-veineuse initialement pratiquée était en usage pour l'hémodialyse depuis au moins 3 mois sans problèmes cliniques majeurs.

La canule classique de dialyse, utilisée pour les canulations artérielle et veineuse, répondait aux caractéristiques suivantes :
14 gauge, 25 mm de longueur et 2 orifices latéraux
et les canules selon l'invention, également pour les canulations artérielle et veineuse, présentaient les caractéristiques ci-dessous :
   Type (a) : 14 gauge, 25 mm de longueur et 2 orifices latéraux.
   Type (b) : 14 gauge, 25 mm de longueur et absence d'orifices latéraux.

### Méthode

Les patients, dont la pression artérielle de départ était de 200 mm Hg ont été dialysés en utilisant la technique à 2 aiguilles, 3 fois par semaine.

Durant 6 semaines de dialyse, la canule classique a été utilisée. Par la suite, ces mêmes patients ont été soumis à 6 autres séances consécutives de dialyse au moyen d'une canule selon l'invention. En conséquence, les patients ont été leurs propres témoins. En outre, uniquement un personnel infirmier ayant 5 ans d'expérience de dialyse a pratiqué les canulations.

D'autre part, les patients ont été invités, au cours de l'étude à évaluer sur une échelle de 0 à 10, la sensation douloureuse enregistrée durant les canulations, ces patients ignorant le type de canule utilisée.

### Résultats

A la suite de ces tests, on a enregistré des débits sanguins respectivement de 430 ml/min et de 400 ml/min.

Par rapport au débit moyen enregistré chez ces patients avec une canule classique, ces tests montrent une augmentation significative de 35 % du débit sanguin à la pression artérielle enregistrée. En outre, aucune différence significative entre les deux types de canule selon l'invention n'a été enregistrée, ce qui tend à montrer que les 2 orifices latéraux de la canule de type (b) se révèlent sans effet notoire sur le débit sanguin.

En outre, la sensation douloureuse enregistrée par les patients lors des canulations s'est avérée plus faible avec les types de canules selon l'invention qu'avec la canule classique.

### 2) Deuxième essai

Un deuxième essai a été entrepris sur un patient en utilisant une canule selon l'invention (14 gauge ; 25 mm de longueur) présentant un biseau distal de 35° comparativement à une canule classique possédant une extrémité distale arrondie.
A cet effet, on a mis en oeuvre la méthode décrite lors du premier essai ci-dessus.

### Résultats

A la suite de ce test, on a enregistré lors de l'utilisation de la canule selon l'invention, des débits sanguins de l'ordre de 500 ml/min par rapport à des débits sanguins de 250 à 300 ml/min enregistrés avec la canule classique.

Ce test montre, par conséquent, une augmentation hautement significative d'au moins 66% du débit sanguin lors de l'utilisation d'une canule selon l'invention par rapport à une canule classique.

D'autre part, la sensation douloureuse lors des canulations s'est révélée plus faible avec la canule selon l'invention qu'avec la canule classique.

L'invention sera mieux comprise et d'autres buts, caractéristiques et avantages de celle-ci apparaîtront plus clairement au cours de la description explicative qui va suivre faite en référence aux dessins schématiques donnés uniquement à titre d'exemples illustrant des modes de réalisation de l'invention et dans lesquels :
- la figure 1 montre une vue en coupe frontale d'une canule d'hémodialyse conforme à l'invention,
- la figure 2 montre une vue frontale en coupe partielle d'un cathéter d'hémodialyse muni de la canule à la figure 1 et logeant une aiguille trocart,
- les figures 3 à 5 montrent respectivement des vues en plan, latérale et frontale du triple biseau d'une aiguille trocart.

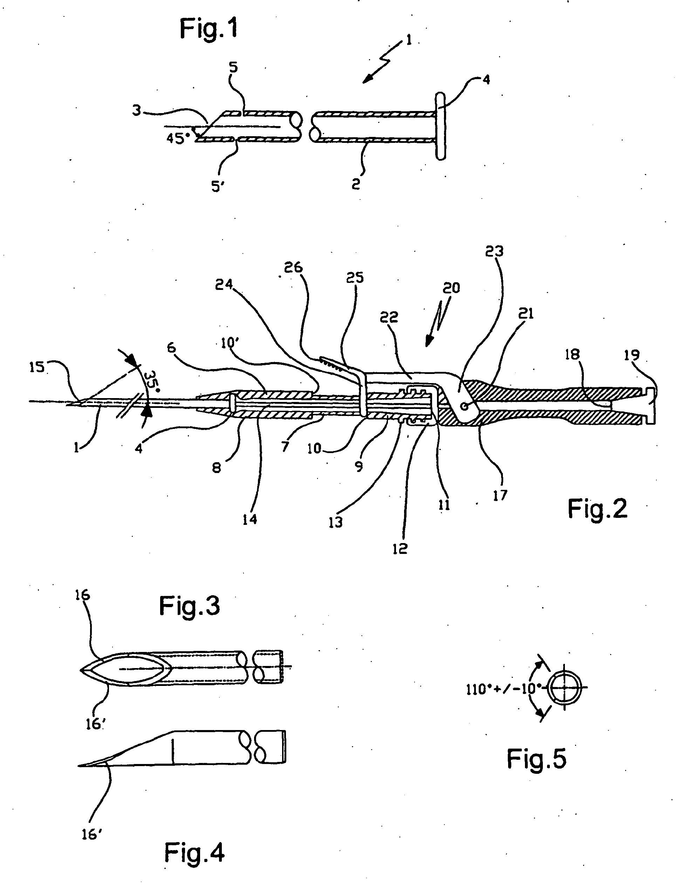

Tel que représenté à la figure 1, la canule 1, formée d'un tube 2 cylindrique en éthylène-propylène fluoré (TEFLON^{®}) présente une extrémité distale taillée en biseau 3 et une embase 4. Dans ce mode de réalisation, le biseau 3 forme un angle de 45° avec l'axe longitudinal de la canule, celle-ci montrant également deux orifices latéraux 5, 5' circulaires placés à proximité de l'extrémité distale en vue d'assurer une régularisation du flux sanguin.

D'autre part, on remarque à la figure 2, un cathéter comprenant la canule 1 dont le biseau 3, dans ce mode de réalisation, forme un angle de 35° avec l'axe longitudinal du tube, et une embase 6, transparente, en polyuréthane, formant chambre de raccord. Cette chambre surmoulée sur l'embase 4 de la canule et partiellement sur la canule elle-même présente une zone de clampage 7 de moindre épaisseur que les extrémités distale 8 et proximale 9 et susceptible de déformation élastique sous l'effet par exemple d'un écrasement.

Comme le montre également la figure 2, cette zone de clampage correspond à un retrait partiel de la chambre 6 de raccord, retrait délimité latéralement par des bords 10 et 10'.

On remarque également, que l'extrémité proximale 9 du cathéter laisse apparaître un orifice 11 muni d'un obturateur 12, avantageusement en polyisoprène, apte à assurer l'étanchéité de la chambre 6. D'autre part, cette même figure 2 montre, à proximité de cet obturateur, des ergots hélicoïdaux 13 capables de coopérer avec la partie femelle correspondante d'une fermeture de type Luer mise en place en particulier sur l'extrémité distale d'une tubulure (non représentée) de liaison à un dialyseur.

En se référant également à la figure 2, on observe une aiguille 14 creuse formant trocart insérée dans la lumière de la canule 1. En outre, l'extrémité distale de cette aiguille, en acier inoxydable siliconé, comporte un triple biseau visible aux figures 3 à 5, notamment un biseau principal 15 formant un angle d'environ 20° par rapport à l'axe longitudinal de l'aiguille.

Moyennant un positionnement approprié de cette aiguille 14 dans la canule 1, les biseaux 3 et 15 peuvent présenter, par conséquent, une configuration telle que leurs angles aigus s'ouvrent dans le même sens comme représenté à la figure 2. On comprend, en conséquence, qu'avant la pose d'une canule d'hémodialyse selon l'invention, celle-ci sera avantageusement positionnée sur l'aiguille trocart biseautée en sorte que l'angle du biseau de la canule et celui du biseau principal de l'aiguille soient ouverts dans le même sens ou sensiblement dans le même sens.

En outre, les bords du biseau 15 comportent des biseaux secondaires 16 et 16' visibles aux figures 3 et 4, ces biseaux latéraux s'éloignant chacun de la pointe de l'aiguille 14 jusqu'à occuper environ 50 % du biseau principal.

D'autre part, on observe également que l'extrémité proximale de l'aiguille débouche à l'intérieur d'une embase 17, ou chambre de visualisation du débit sanguin, par son extrémité distale tandis que l'extrémité proximale de celle-ci comporte un orifice 18 obturé par un bouchon 19 généralement en polypropylène.

L'ensemble formé par le cathéter, logeant l'aiguille 14 assortie de la chambre de visualisation 17 pourvue de son bouchon 19, est également muni d'un système anti-retrait en polypropylène comprenant un clip 20 prenant la forme d'un cavalier solidaire de deux axes de rotation 21 fixés dans la paroi de cette chambre.

Ce cavalier est lui-même formé d'un levier 22 dont chacune des extrémités est occupée par une paire de bras, l'une 23 capable de rotation autour des axes 21, l'autre 24 apte à enserrer la chambre de raccord 6 au niveau de sa zone de clampage 7. Dans ce cas, la paire de bras 24, en butée contre le bord 10 de la zone de clampage, empêche le retrait de l'aiguille logée dans la canule lors d'une traction exercée sur la chambre de visualisation. On remarque, en outre que le levier se prolonge en un appendice 25 de préhension qui, sous l'effet d'une pression exercée par exemple par les doigts d'un utilisateur, permet la rotation du cavalier autour des axes 21 avec, pour conséquence, soit le blocage de l'aiguille dans la canule soit sa désolidarisation offrant la possibilité de son retrait de cette même canule.

De manière à éviter le glissement éventuel du ou des doigts de l'utilisateur lors de cette opération, la face inférieure de cet appendice 25 présente une pluralité de stries ou de saillies 26.

Ce système anti-retrait a, par conséquent, pour avantage de permettre la saisie du cathéter en question par sa partie centrale ou zone de clampage 7 sans risque de retrait de l'aiguille 14 à partir de la lumière de la canule 1 et d'autre part de garantir une qualité de ponction indépendante de la dureté des tissus cutanés traversés.

La canule selon l'invention pour cathéter d'hémodialyse présente des avantages incontestables par rapport à une canule classique, habituellement utilisée dans ce domaine, comportant une extrémité distale arrondie et deux orifices d'amélioration du débit sanguin situés à proximité de cette extrémité.

Ainsi, la canule selon l'invention permet d'obtenir un débit sanguin significativement supérieur et, par conséquent, une durée de la séance de dialyse nettement réduite. Cette supériorité s'est révélée indépendante d'orifices latéraux de proximité d'extrémité distale, ce qui enlève toute nécessité à ces orifices.

D'autre part, une sensation douloureuse moins importante est enregistrée en raison d'un rapprochement du profil de l'extrémité distale de cette canule avec celui de l'aiguille trocart, ce qui assure une transition aiguille/cathéter moins traumatisante lors de la ponction.

## Revendications

1. Canule pour cathéter d'hémodialyse, du genre tube creux cylindrique d'un seul tenant et entièrement en matière souple comprenant une extrémité distale et une extrémité proximale, l'extrémité distale du tube (2) comportant un biseau (3) reliant deux points diamétralement opposés de sa paroi extérieure et formant un angle avec l'axe longitudinal du tube.

2. Canule selon la revendication 1, **caractérisée en ce que** l'angle est compris dans la gamme allant de 35° à 45°.

3. Canule selon la revendication 2, **caractérisée en ce que** l'angle est de 35° ou 45°.

4. Canule selon l'une des revendications 1 à 3, **caractérisée en ce qu'**elle comporte des orifices latéraux (5) situés à proximité de son extrémité distale.

5. Canule selon une des extrémités 1 à 4, **caractérisée en ce que** son extrémité proximale est solidarisée à l'extrémité distale d'une chambre de raccord (6) dont l'extrémité proximale comporte des moyens de verrouillage (13) à une tubulure extérieure ainsi qu'un orifice (11) occupé par un obturateur (12).

6. Canule selon la revendication 5, **caractérisée en ce que** la chambre de raccord est munie d'une branche latérale formant un angle avec celle-ci, cette branche comportant, à son extrémité proximale, des moyens de verrouillage à une tubulure extérieure ainsi qu'un orifice occupé par un obturateur amovible.

7. Canule selon la revendication 5 ou 6, **caractérisée en ce que** la chambre de raccord comporte une zone de clampage (7) capable de déformation élastique de même que la branche latérale lorsqu'elle est présente.

8. Canule selon une des revendications 1 à 7, **caractérisée en ce qu'**elle comporte une aiguille creuse (14) logée dans la lumière du tube.

9. Canule selon la revendication 8, **caractérisée en ce que** l'extrémité distale de l'aiguille comporte un biseau principal (15) reliant deux points diamétralemnet opposés de sa paroi extérieure et formant un angle avec son axe longitudinal.

10. Canule selon une des revendications 1 à 9, **caractérisée en ce que** l'angle aigu formé par le biseau de ladite canule est supérieur à l'angle aigu formé par le biseau principal de l'aiguille.

11. Canule selon une des revendications 1 à 10, **caractérisée en ce que** l'angle aigu formé par le biseau principal de l'aiguille et l'angle aigu formé par le biseau de cette canule sont ouverts dans le même sens ou sensiblement dans le même sens lors de la mise en place de ladite canule dans une fistule d'hémodialyse.

12. Canule selon une des revendications 9 à 11, **caractérisée en ce que** les deux côtés du biseau principal sont pourvus de biseaux latéraux (16 ; 16') secondaires qui délimitent la pointe aiguë de l'aiguille, les faces de ces biseaux latéraux convergeant vers l'intérieur à partir d'un point situé le long de la face du biseau principal jusqu'à un point terminal où leurs plans se rejoignent.

13. Canule selon une des revendications 8 à 12, **caractérisée en ce que** l'extrémité proximale de l'aiguille est solidarisée à l'extrémité distale d'une chambre de visualisation transparente dont l'extrémité proximale comporte un orifice (18) occupé par un bouchon (19) amovible.

14. Canule selon la revendication 13, **caractérisée en ce que** l'extrémité distale de la chambre de visualisation est pourvue d'un moyen anti-retrait (20) capable de coopérer avec la zone de clampage de la chambre de raccord.

## Claims

1. Haemodialysis catheter cannula, of the one-piece cylindrical hollow tube type and entirely made of flexible material comprising a distal end and a proximal end, the distal end of the tube (2) comprising a bevel (3) connecting two diametrically opposed points of the outer wall thereof and forming an angle with the longitudinal tube axis.

2. Cannula according to claim 1, **characterised in that** the angle is within the range from 35° to 45°.

3. Cannula according to claim 2, **characterised in that** the angle is 35° or 45°.

4. Cannula according to any of claims 1 to 3, **characterised in that** it comprises lateral orifices (5) located in the vicinity of the distal end thereof.

5. Cannula according to any of claims 1 to 4, **characterised in that** the proximal end thereof is attached to the distal end of a coupling chamber (6) wherein the proximal end comprises locking means (13) to an outer tube and an orifice (11) occupied by a seal (12).

6. Cannula according to claim 5, **characterised in that** the coupling chamber is equipped with a lateral part forming an angle therewith, said part comprising, at the proximal end thereof, locking means to an outer tube and an orifice occupied by a removable seal.

7. Cannula according to claim 5 or 6, **characterised in that** the coupling chamber comprises a clamping zone (7) capable of elastic deformation similar to the lateral part if it is present.

8. Cannula according to any of claims 1 to 7, **characterised in that** it comprises a hollow needle (14) housed in the tube slot.

9. Cannula according to claim 8, **characterised in that** the distal end of the needle comprises a main bevel (15) connecting two diametrically opposed points of the outer wall thereof and forming an angle with the longitudinal axis thereof.

10. Cannula according to any of claims 1 to 9, **characterised in that** the acute angle formed by the bevel of said cannula is greater than the acute angle formed by the main bevel of the needle.

11. Cannula according to any of claims 1 to 10, **characterised in that** the acute angle formed by the main bevel of the needle and the acute angle formed by the bevel of said cannula are opened in the same direction or substantially in the same direction during the positioning of said cannula in a haemodialysis fistula.

12. Cannula according to any of claims 9 to 11, **characterised in that** both sides of the main bevel are devoid of secondary lateral bevels (16, 16') delimiting the acute tip of the needle, the faces of said lateral bevels converging inwards from a point located along the face of the main bevel to an end-point where the planes thereof join.

13. Cannula according to any of claims 8 to 12, **characterised in that** the proximal end of the needle is attached to the distal end of a transparent viewing chamber wherein the proximal end comprises an orifice (18) occupied by a removable cap (19).

14. Cannula according to claim 13, **characterised in that** the distal end of the viewing chamber is provided with anti-removal means (20) capable of cooperating with the coupling chamber clamping zone.

## Patentansprüche

1. Kanüle für Hämodialysekatheter des Typs eines zylindrischen Hohlkörpers in einem einzigen Stück und vollkommen aus flexiblem Material, umfassend ein distales Ende und ein proximales Ende, wobei das distale Ende der Röhre (2) eine Abschrägung (3) umfasst, die zwei Punkte, diametral entgegengesetzt zu ihrer Außenwand und einen Winkel mit der Längsachse der Röhre bildend, miteinander verbindet.

2. Kanüle nach Anspruch 1, **dadurch gekennzeichnet, dass** der Winkel in einem Bereich von 35° bis 45° liegt.

3. Kanüle nach Anspruch 2, **dadurch gekennzeichnet, dass** der Winkel 35° oder 45° einnimmt.

4. Kanüle nach einem der Ansprüche 1 bis 3, **dadurch gekennzeichnet, dass** sie Seitenschlitze (5) umfasst, die sich in der Nähe ihres distalen Endes befinden.

5. Kanüle nach einem der Enden 1 bis 4, **dadurch gekennzeichnet, dass** ihr proximales Ende fest mit dem distalen Ende einer Verbindungskammer (6) verbunden ist, deren proximales Ende Mittel zur Verriegelung (13) an einen Außenstutzen umfasst, sowie einen Schlitz (11), der von einem Verschluss (12) eingenommen wird.

6. Kanüle nach Anspruch 5, **dadurch gekennzeichnet, dass** die Verbindungskammer mit einem Seitenzweig versehen ist, der einen Winkel mit ihr bildet, wobei dieser Zweig an seinem proximalen Ende Mittel zur Verriegelung an einen Außenstutzen sowie einen Schlitz, der von einem abnehmbaren Verschluss eingenommen wird, umfasst.

7. Kanüle nach Anspruch 5 oder 6, **dadurch gekennzeichnet, dass** die Verbindungskammer eine Klammerzone (7) umfasst, die zur elastischen Verformung fähig ist, sowie der Seitenzweig, wenn dieser vorhanden ist.

8. Kanüle nach einem der Ansprüche 1 bis 7, **dadurch gekennzeichnet, dass** sie eine hohle Nadel (14), die in der Öffnung der Röhre untergebracht ist, umfasst.

9. Kanüle nach Anspruch 8, **dadurch gekennzeichnet, dass** das distale Ende der Nadel eine Haupt-Abschrägung (15) umfasst, die zwei Punkte diametral entgegengesetzt zu ihrer Außenwand und einen Winkel mit ihrer Längsachse bildend miteinander verbindet.

10. Kanüle nach einem der Ansprüche 1 bis 9, **dadurch gekennzeichnet, dass** der spitze Winkel, der durch die Abschrägung von der Kanüle gebildet wird, größer als der spitze Winkel ist, der von der Haupt-Abschrägung der Nadel gebildet wird.

11. Kanüle nach einem der Ansprüche 1 bis 10, **dadurch gekennzeichnet, dass** der spitze Winkel, der von der Haupt-Abschrägung der Nadel gebildet wird, und der spitze Winkel, der von der Abschrägung dieser Kanüle gebildet wird, beim Einführen der Kanüle in eine Hämodialysefistel in derselben Richtung offen sind oder fast in derselben Richtung offen sind.

12. Kanüle nach einem der Ansprüche 9 bis 11, **dadurch gekennzeichnet, dass** die beiden Seiten von der Haupt-Abschrägung mit sekundären Seitenabschrägungen (16 ; 16') versehen sind, die die Spitze der Nadel begrenzen, wobei die Seiten von diesen Seitenabschrägungen nach Innen konvergieren, und dies ab einem Punkt an der Seite der Haupt-Abschrägung entlang bis zum einem Endpunkt, an dem sich ihre Ebenen treffen.

13. Kanüle nach einem der Ansprüche 8 bis 12, **dadurch gekennzeichnet, dass** das proximale Ende der Nadel fest mit dem distalen Ende einer transparenten Sichtkammer verbunden ist, deren proximales Ende einen Schlitz (18) umfasst, der von einem abnehmbaren Stopfen (19) eingenommen wird.

14. Kanüle nach Anspruch 13, **dadurch gekennzeichnet, dass** das distale Ende der Sichtkammer mit einem Anti-Rückzug-Mittel (20) versehen ist, das fähig ist, mit der Klammerzone von der Verbindungskammer zusammenzuwirken.
